# EUROPÄISCHE PATENTSCHRIFT

(11) **EP 4 157 161 B1**
(45) Veröffentlichungstag und Bekanntmachung des Hinweises auf die Patenterteilung: **19.06.2024**
(21) Anmeldenummer: 21729490.9
(22) Anmeldetag: 27.05.2021
(51) Int. Cl.: A61F 4/00

(54) **EINGABEVORRICHTUNG ZUR BEDIENUNG UND/ODER STEUERUNG EINES TECHNISCHEN GERÄTES DURCH EINEN BENUTZER**
INPUT DEVICE FOR OPERATING AND/OR CONTROLLING A TECHNICAL DEVICE VIA A USER
PÉRIPHÉRIQUE D'ENTRÉE POUR L'UTILISATION ET/OU LA COMMANDE D'UN APPAREIL TECHNIQUE PAR UN UTILISATEUR

(30) Priorität: 02.06.2020 DE 102020114632
(43) Veröffentlichungstag der Anmeldung: 05.04.2023
(73) Patentinhaber: Universität Stuttgart, 70174 Stuttgart (DE)
(72) Erfinder: MEHRING, Carsten, 70199 Stuttgart (DE)
(74) Vertreter: Witte, Weller & Partner Patentanwälte mbB
(86) Internationale Anmeldenummer: PCT/EP2021/064258
(87) Internationale Veröffentlichungsnummer: WO 2021/244949

(56) Entgegenhaltungen:
- EP-A1- 0 487 027
- WO-A1-90/07249
- US-A1- 2012 259 554

## Beschreibung

Die vorliegende Erfindung betrifft eine Eingabevorrichtung zur Bedienung und/oder Steuerung eines technischen Gerätes durch einen Benutzer, mit einer Vielzahl von Sensorelementen, die dazu eingerichtet sind, räumlich verteilt in der Mundhöhle des Benutzers angeordnet zu werden, um eine jeweils momentane Zungenposition und/oder Zungenbewegung des Benutzers zu detektieren, und mit einem Schnittstellenschaltkreis, der mit den Sensorelementen gekoppelt ist und der dazu eingerichtet ist, in Abhängigkeit von den Sensorelementen eine Vielzahl von Steuersignalen zu erzeugen und an das technische Gerät zu übertragen.

Die Erfindung betrifft ferner ein Verfahren zur Bedienung und/oder Steuerung eines technischen Gerätes durch einen Benutzer, mit den Schritten: Bereitstellen einer Vielzahl von Sensorelementen räumlich verteilt in der Mundhöhle des Benutzers, Detektieren einer jeweils momentanen Zungenposition und/oder Zungenbewegung des Benutzers unter Verwendung der Sensorelemente, Erzeugen einer Vielzahl von Steuersignalen in Abhängigkeit von der jeweils momentanen Zungenposition und/oder Zungenbewegung des Benutzers mit Hilfe eines Schnittstellenschaltkreises, und Übertragen der Vielzahl von Steuersignalen von dem Schnittstellenschaltkreis an das technische Gerät.

Eine solche Eingabevorrichtung und ein entsprechendes Verfahren sind prinzipiell aus US 2012/0259554 A1 bekannt, die auch als nächstliegender Stand der Technik angesehen werden kann.

Es gibt zahlreiche unterschiedliche Konzepte, um es einem Benutzer zu ermöglichen, mit einem technischen Gerät zu interagieren und insbesondere Daten und/oder Steuerbefehle für das technische Gerät zu generieren. Zur Eingabe von Daten oder Befehlen für einen Computer sind Tastaturen, Mäuse, sogenannte Trackballs und berührungsempfindliche Flächen in Form von Touchscreens oder Touchpads bekannt. Gemeinsam ist diesen Eingabevorrichtungen, dass der Benutzer typischerweise seine Hände oder zumindest einen oder mehrere Finger benötigt, um das technische Gerät zu bedienen. Dementsprechend kann man hier von einer manuellen Eingabevorrichtung sprechen.

Darüber hinaus finden zunehmend auch sprachgesteuerte Eingabevorrichtungen Verwendung. Es gibt jedoch Situationen und Konstellationen, in denen ein Benutzer weder manuell noch sprachgesteuert mit einem technischen Gerät interagieren kann, beispielsweise aufgrund einer körperlichen Behinderung oder aufgrund einer anderen, zeitgleich ausgeführten manuellen Tätigkeit in einer Umgebung, in der eine Spracheingabe unzuverlässig, störend oder sogar verräterisch sein könnte.

Die oben genannte US 2012/0259554 A1 schlägt eine Eingabevorrichtung für einen Computer vor, bei der die Zungenbewegung eines Benutzers innerhalb der Mundhöhle verfolgt wird. In einem ersten Ausführungsbeispiel beinhaltet die Eingabevorrichtung eine magnetische Kugel, die sich innerhalb eines Kugelkäfigs frei drehen kann. Der Kugelkäfig mit der Kugel kann über ein Mundstück in der Mundhöhle des Benutzers platziert werden und der Benutzer kann die Kugel mit seiner Zunge bewegen. Außerhalb der Mundhöhle ist ein magnetischer Sensor vorgesehen, der Änderungen des Magnetfeldes der Kugel detektiert. In einem zweiten Ausführungsbeispiel beinhaltet die Eingabevorrichtung eine Vielzahl von Drucksensoren oder eine Vielzahl von kapazitiven Sensoren, die an einem Mundstück angeordnet sind, welches über die Zähne des Benutzers geschoben werden kann. Vorgeschlagen ist hier, dass die Sensoren sichtbar an der Außen- bzw. Vorderseite der Zähne platziert werden. Die Drucksensoren können einen hinreichenden Druck detektieren, den ein Benutzer mit seiner Zunge erzeugen kann. Kapazitive Sensoren können die Nähe der Zunge auch ohne Berührung detektieren. Die Signale der Sensoren sollen drahtlos an einen Prozessor übertragen werden, der insoweit als Schnittstellenschaltkreis zu einem Computer dienen kann. Die erforderlichen Funksignale sollen hier in Reaktion auf eine bestimmte Zungenbewegung des Benutzers von den Sensorelementen erzeugt werden. Dies deutet darauf hin, dass die Sensoren eine im Details nicht dargestellte Stromversorgung benötigen. In einem dritten Ausführungsbeispiel schlägt US 2012/0259554 A1 vor, dass die Zungenbewegung des Benutzers mit Hilfe einer in der Mundhöhle angeordneten thermischen Kamera verfolgt wird. Weitere Ausführungsbeispiele beinhalten Mikrofone außerhalb der Mundhöhle, mit denen die Zungenbewegung verfolgt werden soll. Insgesamt schlägt US 2012/0259554 A1 somit eine Vielzahl von Konzepten vor, die es einem Benutzer ermöglichen sollen, ein technisches Gerät mit der Zunge zu bedienen und/oder zu steuern.

Eine weitere Eingabevorrichtung zur Bedienung und/oder Steuerung eines technischen Gerätes mit Hilfe der Zunge ist in US 2016/0154468 A1 offenbart. In einem ersten Ausführungsbeispiel beinhaltet die Vorrichtung einen Stab mit integrierten Sensorelementen, der beispielsweise durch ein Piercing an der Zunge des Benutzers befestigt wird. Die Sensorelemente in dem Stab können einen Drehratensensor, einen Drucksensor und/oder einen berührungsempfindlichen Sensor beinhalten, so dass der Benutzer durch Anlegen der Zunge an den Gaumen oder andere Zungenbewegungen mit einem technischen Gerät interagieren kann. In einem weiteren Ausführungsbeispiel kann ein berührungsempfindliches Sensorelement als Zahnimplantat in der Mundhöhle des Benutzers angeordnet werden.

Noch eine weitere Eingabevorrichtung zur Bedienung und/oder Steuerung eines technischen Gerätes mit Hilfe der Zunge ist in US 7,995,031 B2 offenbart. In diesem Fall beinhaltet die Eingabevorrichtung Drucksensoren, die unter dem Gaumen angeordnet werden. Eine weitere Eingabevorrichtung mit Drucksensoren ist in US 8,242,880 B2 offenbart. US 8,018,320 B2 schlägt eine zungengesteuerte Eingabevorrichtung vor, die auf Induktion basiert. US 8,548,394 B2 offenbart eine zungengesteuerte Eingabevorrichtung, die unter dem Gaumen angeordnet wird und auf Induktion, auf einer mechanischen Interaktion oder auf einer berührungsempfindlichen Fläche beruht.

Aus US 2018/0000563 A1 ist ein Funkmodul bekannt, das dazu dient, eine Zahnregulierung im Rahmen einer kieferorthopädischen Behandlung zu überwachen. Des Weiteren kann das Funkmodul verwendet werden, um Parameter bezüglich der Atmung und des Schlafs einer Person zu überwachen. Das Funkmodul kann mit Hilfe eines Trägers an den Zähnen eines Patienten befestigt werden.

WO 2006/073296 A1 offenbart einen RFID-Tag zur Befestigung an einem menschlichen Zahn. Der RFID-Tag beinhaltet einen Speicher zum Abspeichern von persönlichen Informationen, die den Träger insbesondere bei einem Unfall identifizieren sollen.

US 8,487,769 B2 offenbart einen schaltbaren RFID-Transponder. US 2013/090921 A1 offenbart eine Eingabevorrichtung zur Spracheingabe.

WO 90/07249 A1 offenbart einen durch die Zunge aktivierten Kommunikationscontroller mit einer intraoralen Senderbaugruppe mit einer Tastatur, die mehrere durch die Zunge aktivierbare Positionen zum Kodieren eines Signals besitzt. Die intraorale Senderbaugruppe umfasst eine zweiseitige Leiterplatte. Die erste Seite enthält Elektronik zum Übertragen von Signalen und die zweite Seite enthält Schaltkreise zum Umschalten von einer Position in eine andere. Der Controller umfasst außerdem einen Empfänger zum Empfang der codierten Signale vom Sender. Der Empfänger sendet das Signal an einen Mikrocomputer zur Decodierung der codierten Signale, um aus dem decodierten Signal einen Befehlssatz zum Betreiben eines Geräts zu bilden. Der Mikrocomputer sendet die Signale an ein zu bedienendes Gerät.

EP 0 487 027 A1 offenbart eine Bedienungsvorrichtung für körperbehinderte Personen mit einem Schaltgerät, das aus einer Platine mit einem Tastenfeld besteht. Die Platine kann beispielsweise als Kaugummibrett im Mund positioniert werden, so dass das Tastenfeld mit der Zunge betätigt werden kann. Auf diese Weise ausgelöste Signale werden an eine Schnittstelle übertragen, über die verschiedene Geräte bedient oder gesteuert werden können.

Vor diesem Hintergrund ist es eine Aufgabe der vorliegenden Erfindung, eine alternative Eingabevorrichtung anzugeben, die eine zuverlässige Bedienung und/oder Steuerung eines technischen Gerätes mit Hilfe der Zunge ermöglicht. Es ist insbesondere eine Aufgabe der Erfindung, eine Eingabevorrichtung anzugeben, die eine große Vielfalt an Benutzereingaben ermöglicht und Fehleingaben möglichst weitgehend reduziert. Es ist eine Eingabevorrichtung wünschenswert, die eine für den Benutzer komfortable und unbedenkliche/ungefährliche Interaktion mit einem technischen Gerät mit Hilfe der Zunge ermöglicht.

Die Aufgaben werden durch eine Eingabevorrichtung der eingangs genannten Art gelöst, wobei die Sensorelemente jeweils einen Transponder beinhalten, der dazu eingerichtet ist, ein Abfragesignal aufzunehmen und in Abhängigkeit davon ein individuell codiertes Antwortsignal mit einer individuellen Codierung an den Schnittstellenschaltkreis zu übertragen, wobei der Schnittstellenschaltkreis dazu eingerichtet ist, das Abfragesignal zu erzeugen und über eine Antenne in die Mundhöhle des Benutzers auszusenden, und wobei der Schnittstellenschaltkreis aufgrund der individuellen Codierung erkennen kann, von welchem der Sensorelemente in der Mundhöhle des Benutzers ein jeweiliges Antwortsignal stammt. Des Weiteren wir ein Verfahren der eingangs genannten Art vorgeschlagen, wobei der Schnittstellenschaltkreis ein Abfragesignal erzeugt und über eine Antenne in die Mundhöhle des Benutzers aussendet, und wobei die Sensorelemente jeweils einen Transponder beinhalten, der das Abfragesignal aufnimmt und ein individuell codiertes Antwortsignal mit einer individuellen Codierung in Abhängigkeit von der jeweils momentanen Zungenposition und/oder Zungenbewegung des Benutzers an den Schnittstellenschaltkreis überträgt, wobei der Schnittstellenschaltkreis aufgrund der individuellen Codierung erkennen kann, von welchem der Sensorelemente in der Mundhöhle des Benutzers ein jeweiliges Antwortsignal stammt.

In bevorzugten Ausführungsbeispielen beinhalten die Sensorelemente jeweils einen passiven Transponder, d.h. einen Transponder, der die zum Erzeugen des individuell codierten Antwortsignals benötigte Sendeenergie aus dem Abfragesignal generiert bzw. mit dem Abfragesignal erhält. Dementsprechend kommen die Sensorelemente in den bevorzugten Ausführungsbeispielen ohne eigenen Energiespeicher aus. Insbesondere kann auf eine Batterie oder einen wieder aufladbaren Akkumulator in der Mundhöhle verzichtet werden, was zur Vermeidung von gesundheitlichen Beeinträchtigungen von großem Vorteil ist.

Ungeachtet dessen könnte die Eingabevorrichtung jedoch in anderen Ausführungsbeispielen einen Energiespeicher in Form einer Batterie, eines Akkus oder einer Kapazität, etwa in Form eines sogenannten Supercaps, besitzen, wobei der Energiespeicher zusammen mit den Sensorelementen in der Mundhöhle des Benutzers angeordnet werden kann. Vorteilhaft kann ein solcher Energiespeicher berührungslos geladen werden, insbesondere induktiv oder kapazitiv. Prinzipiell kann ein solcher Energiespeicher auch mit Hilfe des sogenannten "Energie-Harvesting" durch die Zungenbewegung selbst geladen werden.

Jedenfalls besitzen die Sensorelemente der neuen Eingabevorrichtung jeweils einen Transponder, der in der Lage ist, ein individuell codiertes Antwortsignal an den Schnittstellenschaltkreis zu übertragen. Jedes Sensorelement besitzt also "einen eigenen" Transponder, der das Sensorelement identifiziert. Infolgedessen kann der Schnittstellenschaltkreis auf einfache und zuverlässige Weise erkennen, von welchem Sensorelement aus der Vielzahl von Sensorelemente ein jeweiliges Antwortsignal stammt. Dies macht es möglich, die jeweils aktuelle Position und/oder Bewegung der Zunge mit einer hohen Ortsauflösung und einer hohen Zuverlässigkeit zu erkennen.

Insbesondere ist es mit der neuen Eingabevorrichtung auf sehr einfache und kostengünstige Weise möglich, eine hohe Anzahl an Zähnen mit jeweils einem oder sogar mehreren Sensorelementen zu versehen, um auf diese Weise eine hohe Anzahl an "Tasten" zur komfortablen und genauen zungengesteuerten Eingabe von Daten und/oder Steuerbefehlen zu realisieren. Die menschliche Zunge kann mit einer hohen Spezifizität und Ortsauflösung die Zähne in der Mundhöhle des Benutzers lokalisieren. Zudem kann die menschliche Zunge schon kleinste Erhebungen, Vertiefungen und Veränderungen in der Mundhöhle detektieren. Die neue Eingabevorrichtung macht sich diese Eigenschaft in vorteilhafter Weise zunutze. Prinzipiell könnten an jedem Zahn des Benutzers ein oder sogar mehrere Sensorelemente angeordnet werden, etwa an verschiedenen Seiten eines Zahns einschließlich der zur Zunge hin weisenden Zahninnenseite, der von der Zunge weg weisenden Zahnaußenseite sowie ggf. den Kauflächen. Damit eröffnet die neue Eingabevorrichtung eine große Vielzahl an Daten- und/oder Befehlseingaben, die aufgrund der jeweils individuellen Codierung der Transponder sehr zuverlässig voneinander unterschieden werden können, ohne dass es einer aufwendigen Auswerteschaltung in der Mundhöhle des Benutzers bedürfte. Prinzipiell ist es mit der neuen Eingabevorrichtung möglich, die Belegung einer herkömmlichen Computertastatur mit einem entsprechenden ASCII-Zeichensatz zu implementieren.

Eine Bewegung der Zunge entlang der Zahninnenseiten ermöglicht einem Benutzer eine sehr komfortable und ermüdungsfreie Interaktion. Insgesamt kann mit der neuen Eingabevorrichtung daher eine komfortable, zuverlässige und sichere Interaktion eines Benutzers mit einem technischen Gerät realisiert werden. Die oben genannten Aufgaben sind daher vollständig gelöst.

In einer bevorzugten Ausgestaltung sind die Sensorelemente jeweils an einem Sensorträger angeordnet, der dazu eingerichtet ist, die Sensorelemente an den Zähnen des Benutzers zu platzieren, insbesondere an den Zahninnenseiten, die zur Zunge weisen.

Abweichend hiervon oder ergänzend hierzu könnten die Sensorelemente in anderen Ausgestaltungen an anderen Stellen innerhalb der Mundhöhle eines Benutzers platziert sein, beispielsweise am Gaumen oder an den Kieferknochen. Die Platzierung der Sensorelemente an den Zähnen trägt vorteilhaft dazu bei, eine fehlerfreie und komfortable Interaktion mit dem technischen Gerät zu ermöglichen, weil die Zähne eine für den Benutzer bekannte und gewohnte Referenzposition repräsentieren. Besonders vorteilhaft ist es, die einzelnen Sensorelemente jeweils an Zahninnenseiten zu platzieren, weil der Benutzer die Sensorelemente dann mit sehr geringen feinmotorischen Bewegungen erreichen kann. In einigen Ausführungsbeispielen kann der Sensorträger jeweils ein einzelnes Sensorelement an einem Zahn halten. In anderen Ausführungsbeispielen kann der Sensorträger mehrere Sensorelemente halten, beispielsweise nach Art einer Brücke und/oder als Formmodell, das als Negativabdruck an die Außenkontur eines oder mehrerer Zähne angepasst ist. In einigen Ausführungsbeispielen kann der Sensorträger einen Silikonkörper beinhalten, der nach Art eines Zahnschutzes oder einer Zahnschiene über die Zähne des Benutzers geschoben werden kann. In anderen Ausführungsbeispielen können die Sensorelemente auf Zahnflächen aufgeklebt sein, so dass in diesen Fällen eine Gehäusewand des jeweiligen Sensorelements, die mit Hilfe eines körperverträglichen Klebstoffs an einem oder mehreren Zähnen des Benutzers fixiert werden kann, den Sensorträger bildet oder beinhaltet. Die Positionierung dieser Sensorelemente kann vorteilhaft mit Hilfe eines Applikators erfolgen. Mehrere Sensorelemente können gruppenweise an einem gemeinsamen Halter angeordnet sein. In einigen Ausführungsbeispielen kann die Eingabevorrichtung Sensorträger beinhalten, die jeweils zwei Sensorelemente pro Zahn halten können.

In einer weiteren Ausgestaltung beinhaltet die Eingabevorrichtung einen Halter, der dazu eingerichtet ist, den Schnittstellenschaltkreis außerhalb der Mundhöhle des Benutzers zu halten.

Vorzugsweise ist der Halter dazu eingerichtet, den Schnittstellenschaltkreis im Kopf- und/oder Halsbereich des Benutzers zu halten. In einigen Ausführungsbeispielen kann der Halter einen Bügel aufweisen, der im bestimmungsgemäßen Einsatz im Wangenbereich des Benutzers angeordnet ist und beispielsweise nach Art eines Mikrofon-Headsets am Kopf und/oder Ohr des Benutzers befestigt werden kann. In weiteren Ausführungsbeispielen kann der Halter nach Art eines Brillengestells vor dem Gesicht des Benutzers angeordnet sein oder an einem Halsband oder einer Halskette um den Hals des Benutzers gelegt sein. In weiteren Ausführungsbeispielen kann der Schnittstellenschaltkreis auf einer flexiblen, auf der Haut haftenden Folie bzw. als sogenanntes elektronisches Tattoo realisiert sein, die bzw. das im Wangen-, Kinn- und/oder Halsbereich des Benutzers positioniert ist. All diese Ausgestaltungen und Ausführungsbeispiele besitzen den Vorteil, dass die Übertragung der individuellen Codierung sowie der wechselseitige Austausch von Abfragesignal und Antwortsignal über kurze Distanzen ermöglicht wird. Dies erlaubt es, die neue Eingabevorrichtung mit sehr geringen Sendeleistungen zu realisieren, was sowohl für die Betriebsdauer als auch für die Gesundheit des Benutzers vorteilhaft erscheint.

In einer weiteren Ausgestaltung beinhalten die Sensorelemente jeweils einen SAW-Tag als Transponder.

Die Abkürzung SAW steht hier für Oberflächenwelle (Surface Acoustic Wave) und bezeichnet eine Schallwelle, die sich an der Oberfläche eines Körpers ausbreitet. Die Sensorelemente beinhalten in dieser Ausgestaltung einen Wellenwandler (typischerweise als Interdigitalwandler) , der ein elektrisches, magnetisches und/oder elektromagnetisches Abfragesignal in eine mechanische Schallwelle umwandelt. Die Schallwelle kann sich an der Oberfläche des Trägerkörpers ausbreiten und wird an definierten Reflexionsstellen reflektiert. Eine so reflektierte Schallwelle läuft an der Oberfläche des Körpers zurück und wird von dem Wellenwandler als Antwortsignal abgestrahlt. In bevorzugten Ausführungsbeispielen besitzt jedes Sensorelement einen SAW-Tag mit einer individuellen Codierung in Form einer räumlichen Verteilung von Reflexionsstellen, so dass das individuell codierte Antwortsignal jeweils eine Vielzahl von Reflexionen beinhaltet, deren zeitlicher Abstand relativ zu dem Abfragesignal und relativ zueinander die individuelle Codierung repräsentiert.

Die Verwendung derartiger SAW-Tags ermöglicht eine sehr kostengünstige und zuverlässige Identifikation der einzelnen Sensorelemente. Die Schallwelle an der Oberfläche des Sensorelements breitet sich langsamer aus als eine elektromagnetische Funkwelle, was prinzipbedingt zu einer zeitlichen Verzögerung zwischen Abfragesignal und den individuell codierten Antwortsignalen der Sensorelemente führt. Diese zeitliche Verzögerung ist sehr vorteilhaft, um eine Vielzahl von individuell codierten Antwortsignalen in Reaktion auf ein gemeinsames Abfragesignal auszuwerten, insbesondere in der Präsenz von natürlichen Reflexionen des Abfragesignals in der Mundhöhle des Benutzers. Die Verzögerung reduziert zudem ein direktes Übersprechen vom Abfragesignal zum Antwortsignal am Schnittstellenschaltkreis. Darüber hinaus können SAW-Tags im Gegensatz zu vielen anderen Sensorelementen mit sehr kleinen Abmessungen von beispielsweise kleiner oder gleich 2mm x 2mm x 0,2mm hergestellt werden. Dies begünstigt eine Positionierung direkt an den Zähnen des Benutzers. Die SAW-Tags können in einigen Ausführungsbeispielen mit einer körperverträglichen Beschichtung versehen und mit Hilfe eines körperverträglichen Klebstoffs an den Zähnen des Benutzers befestigt werden. Vorteilhaft können derartige Sensorelemente dann durch einfaches Zähneputzen entfernt und beispielsweise über den Darmtrakt ausgeschieden werden. Dies reduziert oder vermeidet Hygieneprobleme.

In einer weiteren Ausgestaltung ist der Schnittstellenschaltkreis dazu eingerichtet, eine Vielzahl von individuell codierten Antwortsignalen in Bezug auf eine jeweilige Signalstärke zu analysieren.

In dieser Ausgestaltung beinhaltet die Auswertung der Sensorelemente eine Analyse der jeweiligen Signalstärke in Ergänzung dazu, dass der Schnittstellenschaltkreis die jeweils individuelle Codierung der Sensorelemente analysiert. Die Analyse der jeweiligen Signalstärke kann in einigen Ausführungsbeispielen unter Verwendung eines Schwellenwertkriteriums erfolgen, indem der Schnittstellenschaltkreis zunächst anhand eines Schwellenwertkriteriums entscheidet, welche Antwortsignale aus der Vielzahl von Antwortsignalen der Sensorelemente einer weiteren Analyse/Auswertung zugeführt werden. In einigen bevorzugten Ausführungsbeispielen ist der Schnittstellenschaltkreis dazu eingerichtet, nur solche individuell codierten Antwortsignale in Bezug auf den jeweils individuellen Code zu analysieren, deren Signalstärke einen definierten Schwellenwert übersteigt oder zumindest erreicht. Die Ausgestaltung ermöglicht die Realisierung der neuen Eingabevorrichtung auf sehr einfache und kostengünstige Weise mit rein passiven Sensorelementen, da die Zunge einen Signalaustausch zwischen einem Sensorelement und dem Schnittstellenschaltkreis dämpft oder gar unterdrückt, wenn sie das Sensorelement abdeckt. Dementsprechend kann die Eingabevorrichtung dieser Ausgestaltung darauf beruhen, dass der Benutzer mit Hilfe seiner Zunge einen Signalaustausch zwischen dem Schnittstellenschaltkreis und einem oder mehreren ausgewählten Sensorelementen gezielt dämpfen und/oder unterdrücken kann. Vorteilhaft kann in dem Schnittstellenschaltkreis hinterlegt sein, welche individuell codierten Antwortsignale der Schnittstellenschaltkreis in Reaktion auf ein Abfragesignal erwarten kann, wenn keines der Sensorelemente oder nur bestimmte Sensorelemente vom Benutzer mit seiner Zunge abgedeckt sind. Der Schnittstellenschaltkreis kann dazu eingerichtet sein, die empfangenen Antwortsignale mit der Erwartungshaltung zu vergleichen, um auf diese Weise zu erkennen, von welchem Sensorelement oder von welchen Sensorelementen kein Antwortsignal mit hinreichender Signalstärke empfangen wurde. Auf diese Weise kann der Schnittstellenschaltkreis sehr einfach die "Betätigung" eines Sensorelements mit der Zunge, genau genommen eine gewollte Assoziation zwischen Zunge und Zahn oder Zähnen, erkennen.

In einer weiteren Ausgestaltung ist der Schnittstellenschaltkreis dazu eingerichtet, eine Vielzahl von individuell codierten Antwortsignalen logisch miteinander zu verknüpfen.

Diese Ausgestaltung trägt vorteilhaft dazu bei, dass der Schnittstellenschaltkreis zeitgleiche und/oder zeitlich aufeinanderfolgende "Betätigungen" einer Gruppe von Sensorelementen zuverlässig erkennen und auswerten kann. Besonders vorteilhaft ist der Schnittstellenschaltkreis in einigen Ausführungsbeispielen dieser Ausgestaltung dazu eingerichtet, eine Zungenbewegung entlang von mehreren Sensorelementen in Bezug auf Bewegungsrichtung und/oder Bewegungsgeschwindigkeit zu detektieren. Dies ermöglicht es dem Benutzer, zum Beispiel über "Wischbewegungen" ähnlich wie auf einem berührungsempfindlichen Bildschirm eines Smartphones oder Tablet-PCs mit dem technischen Gerät zu interagieren. Beispielsweise könnte eine Bewegung mit der Zunge von links nach rechts entlang der Innenseite der Zähne im Oberkiefer des Benutzers als ein Eingabebefehl für ein technisches Gerät fungieren, das eine Bewegung des Gerätes nach rechts auslöst. Das technische Gerät kann beispielsweise ein assistives Robotersystem sein. Auch das Erkennen und Ignorieren von eventuell unbeabsichtigten Mehrfachbetätigungen und eine daraus folgende Erhöhung der Zuverlässigkeit der neuen Eingabevorrichtung wird mit dieser Ausgestaltung erleichtert.

In einer weiteren Ausgestaltung beinhalten die Sensorelemente jeweils einen Schalter, der dazu eingerichtet ist, den Transponder wahlweise zu aktivieren oder zu deaktivieren.

In einigen Ausführungsbeispielen dieser Ausgestaltung kann der Schalter dazu eingerichtet sein, eine elektrische Verbindung zwischen einem ersten Teil des Sensorelements und einem zweiten Teil des Sensorelements wahlweise herzustellen oder zu unterbrechen, wobei der erste Teil des Sensorelements eine Antenne beinhaltet, über die das Abfragesignal aufgenommen und/oder das Antwortsignal abgesendet wird, während der zweite Teil die individuelle Codierung des jeweiligen Sensorelements speichert oder repräsentiert. In einigen Ausführungsbeispielen kann der Schalter ein elektrischer Kontakt sein, der wahlweise mit der Zunge geschlossen werden kann. Insbesondere kann der Schalter einen Wechselkontakt beinhalten, mit dem ein erstes Sensorelement oder ein zweites Sensorelement wahlweise aktiviert werden kann.

Diese Ausgestaltung ist besonders vorteilhaft, wenn der Schnittstellenschaltkreis jeweils nur von solchen Sensorelementen ein individuell codiertes Antwortsignal erhalten soll, die durch eine Benutzeraktion ausgewählt werden. Die Ausgestaltung ermöglicht eine schnelle Erkennung eines vom Benutzer mit der Zunge aktivierten Sensorelements. Sie trägt außerdem dazu bei, dem Benutzer ein haptisches Feedback für die Betätigung eines Sensorelements zu geben. Dies kann vorteilhaft sein, um Fehleingaben zu reduzieren.

In einer weiteren Ausgestaltung weisen die Sensorelemente jeweils eine definierte Kontaktfläche für die Zunge des Benutzers auf, wobei der Schalter senkrecht zu der Kontaktfläche betätigt werden kann.

In dieser Ausgestaltung können die Sensorelemente nach Art eines Drucktasters agieren, was eine sehr zuverlässige individuelle Betätigung einzelner Sensorelemente mit der Zunge ermöglicht.

In einer weiteren Ausgestaltung weisen die Sensorelemente jeweils eine definierte Kontaktfläche für die Zunge des Benutzers auf, wobei der Schalter parallel zu der Kontaktfläche betätigt werden kann.

In dieser Ausgestaltung erfordert die Aktivierung eines Sensorelements gewissermaßen eine Schiebebetätigung des Schalters mit der Zunge bzw. eine mit der Zunge ausgeübte Scherkraft, was vorteilhaft dazu beiträgt, Fehleingaben zu reduzieren. Darüber hinaus ist diese Ausgestaltung vorteilhaft, wenn ein Sensorelement wahlweise zwei verschiedene Antwortsignale erzeugen soll, um die Anzahl der möglichen Interaktionen des Benutzers mit dem technischen Gerät weiter zu erhöhen.

In einer weiteren Ausgestaltung weisen die Sensorelemente jeweils eine Erhebung und/oder Vertiefung auf, die der Zunge des Benutzers eine tastbare Markierung bietet.

Diese Ausgestaltung trägt vorteilhaft dazu bei, die Ergonomie der neuen Eingabevorrichtung zu verbessern und unbeabsichtigte Fehleingaben zu minimieren.

In einer weiteren Ausgestaltung beinhaltet die Eingabevorrichtung einen Kieferpositionssensor, der dazu eingerichtet ist, eine Kieferposition des Benutzers zu detektieren, wobei der Schnittstellenschaltkreis dazu eingerichtet ist, die Vielzahl von Steuersignalen ferner in Abhängigkeit von dem Kieferpositionssensor zu erzeugen.

Diese Ausgestaltung trägt vorteilhaft dazu bei, die Anzahl möglicher Benutzereingaben weiter zu erhöhen. In einigen bevorzugten Ausführungsbeispielen kann der Kieferpositionssensor nach Art einer Shift- bzw. Umschalttaste fungieren, so dass der Benutzer in Ergänzung zu einer Zungenbewegung durch Öffnen oder Schließen seines Kiefers verschiedene Interaktionen mit dem technischen Gerät bewirken kann. In bevorzugten Ausführungsbeispielen ist der Schnittstellenschaltkreis dementsprechend dazu eingerichtet, in Abhängigkeit von einem Signal des Kieferpositionssensors verschiedene Steuersignale zu einem individuell codierten Antwortsignal eines ausgewählten Sensorelements zu erzeugen. In einigen Ausführungsbeispielen kann der Kieferpositionssensor dazu eingerichtet sein, einen Öffnungswinkel zwischen dem Oberkiefer und dem Unterkiefer des Benutzers zu detektieren. In weiteren Ausführungsbeispielen kann der Kieferpositionssensor dazu eingerichtet sein, ein druckabhängiges Kieferpositionssignal zu erzeugen, das beispielsweise für den Druck repräsentativ sein kann, mit dem der Oberkiefer und der Unterkiefer gegeneinandergedrückt werden.

In einer weiteren Ausgestaltung weisen die Sensorelemente jeweils eine geschlossene biokompatible Hülle auf.

Eine biokompatible Hülle im Sinne dieser Ausgestaltung ist eine geschlossene Hülle aus einem Material, das im Kontakt mit dem Speichel und/oder Gewebe des Benutzers frei von gesundheitsschädlichen Einflüssen ist. In einigen Ausführungsbeispielen können die Sensorelemente mit Proteinen überzogen sein, um eine Biokompatibilität zu erreichen. Die Sensorelemente dieser Ausgestaltung besitzen eine hohe Körperverträglichkeit und sind vorzugsweise biotolerant oder sogar bioinert.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden in der nachfolgenden Beschreibung näher erläutert. Es zeigen:
- Fig. 1: eine schematische Darstellung eines ersten Ausführungsbeispiels der neuen Vorrichtung,
- Fig. 2: eine schematische Darstellung eines SAW-Tags, der in dem Ausführungsbeispiel gemäß Fig. 1 als Sensorelement verwendet sein kann,
- Fig. 3: eine schematische Darstellung eines weiteren Ausführungsbeispiels mit mehreren Sensorelementen,
- Fig. 4: eine schematische Darstellung eines Sensorelements gemäß einem weiteren Ausführungsbeispiel, und
- Fig. 5: eine schematische Darstellung eines Sensorelements gemäß einem noch weiteren Ausführungsbeispiel.

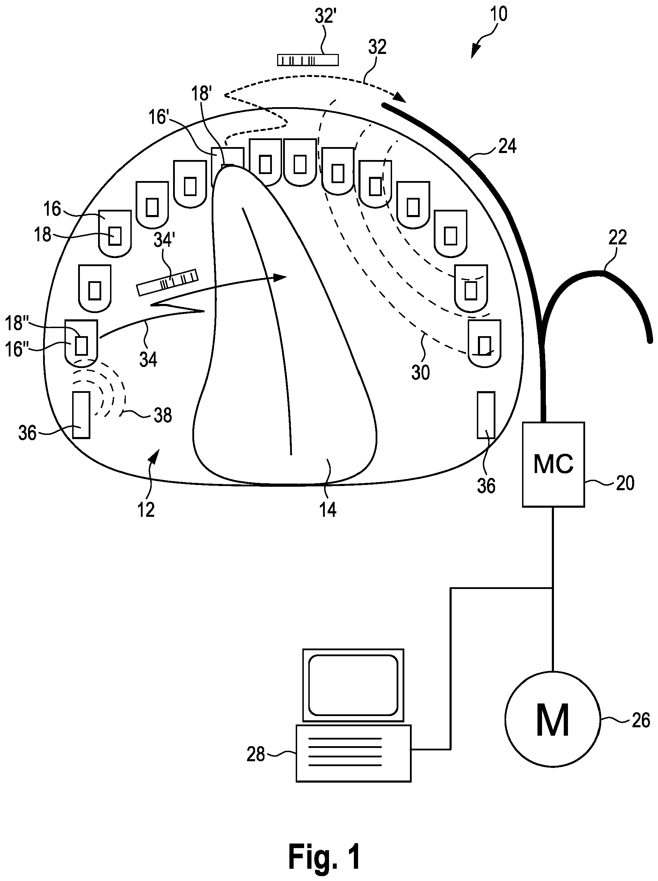

In Fig. 1 ist ein Ausführungsbeispiel der neuen Eingabevorrichtung in seiner Gesamtheit mit der Bezugsziffer 10 bezeichnet. Die Eingabevorrichtung 10 dient dazu, einem menschlichen Benutzer die Interaktion mit einem technischen Gerät zu ermöglichen, wie dies im Einzelnen nachfolgend erläutert ist. Der Benutzer ist hier stark vereinfacht mit seiner Mundhöhle 12 dargestellt, in der sich die Zunge 14 sowie eine Vielzahl von Zähnen 16 befinden. In dem dargestellten Ausführungsbeispiel ist an den Zähnen 16 jeweils ein Sensorelement 18 an einer der Zunge 14 zugewandten Zahninnenseite angeordnet. In einigen Ausführungsbeispielen weisen die Sensorelemente 18 jeweils eine geschlossene Außenhülle auf und sind mit dieser Außenhülle an jeweils einem Zahn festgeklebt. In anderen Ausführungsbeispielen können die Sensorelemente an einem hier nicht dargestellten Sensorträger befestigt sein, wobei der Sensorträger an den Zähnen des Benutzers befestigt wird. In einigen Ausführungsbeispielen kann der Sensorträger ähnlich wie eine Zahnspange an den Zähnen des Benutzers befestigt sein. Alternativ kann der Sensorträger eine Schiene aus Kunststoff mit einer Negativform der Zähne beinhalten, die über die Zähne geschoben werden kann. Unabhängig von der Art der Befestigung der Sensorelemente an den Zähnen des Benutzers ist es in einigen Ausführungsbeispielen bevorzugt, wenn innerhalb der Mundhöhle zumindest sechs diskrete Sensorelemente verteilt sind, wobei jeweils ein diskretes Sensorelement an einem anderen Zahn angeordnet ist.

Die Eingabevorrichtung 10 beinhaltet einen Schnittstellenschaltkreis 20, der in bevorzugten Ausführungsbeispielen einen Mikrocontroller, Mikroprozessor, ASIC, FPGA oder einen anderen Logikschaltkreis beinhalten kann. Der Schnittstellenschaltkreis wird in den bevorzugten Ausführungsbeispielen außerhalb von der Mundhöhle des Benutzers gehalten. Beispielsweise kann der Schnittstellenschaltkreis 20 mit Hilfe eines Bügels 22 gehalten werden, der nach Art eines Headsets an einem oder beiden Ohren des Benutzers befestigt wird. In weiteren Ausführungsbeispielen kann der Schnittstellenschaltkreis 20 an einer Kette oder einem Halsband angeordnet sein, die/das der Benutzer um den Hals tragen kann. In weiteren Ausführungsbeispielen kann der Schnittstellenschaltkreis 20 an einer Brille oder einem brillenartigen Gestell, einem Helm oder einer ähnlichen Halterung angeordnet sein, die es einem Benutzer ermöglicht, den Schnittstellenschaltkreis 20 im Kopfbereich zu positionieren. In bevorzugten Ausführungsbeispielen besitzt der Schnittstellenschaltkreis 20 eine Antenne 24, die sich beispielsweise bügelartig entlang des Kieferknochens und/oder einer Wangenseite des Benutzers erstrecken kann oder als elektronisches Tattoo direkt auf der Haut des Benutzers angebracht ist.

In dem hier dargestellten Ausführungsbeispiel ist der Schnittstellenschaltkreis 20 mit einem elektrischen Antrieb 26 und einem Computer 28 verbunden. Der elektrische Antrieb 26 und der Computer 28 sind Beispiele für ein technisches Gerät, das der Benutzer mit Hilfe der neuen Eingabevorrichtung 10 bedienen und/oder steuern kann. Beispielsweise können der Antrieb 26 und der Computer 28 Teile eines assistiven Robotersystems sein. Generell kann die Eingabevorrichtung 10 überall eingesetzt werden, wo eine manuelle oder sprachbasierte Daten- und/oder Befehlseingabe für ein technisches Gerät nicht, nur zeitweise oder nur unzureichend möglich ist, insbesondere im Bereich des Gesundheitswesens, bei militärischen Anwendungen wie zum Beispiel der Justierung eines Zielfernrohrs im Anschlag oder im Freizeit- und Sportbereich, etwa bei sogenannten Actionsportarten. In einigen Ausführungsbeispielen kann der Schnittstellenschaltkreis 20 wahlweise mit mehreren technischen Geräten gekoppelt werden, die räumlich entfernt voneinander positioniert sind, etwa in verschiedenen Räumen eines Gebäudes. Der Benutzer kann dann die verschiedenen technischen Geräte mit ein und derselben Eingabevorrichtung 10 bedienen und/oder steuern, jeweils abhängig davon, zu welchem der verschiedenen technischen Geräte er sich zu einem definierten Zeitpunkt in der Nähe befindet. Dementsprechend kann der Schnittstellenschaltkreis 20 in bevorzugten Ausführungsbeispielen eine Funkschnittstelle zu dem technischen Gerät aufweisen, die beispielsweise als WLAN und/oder Bluetooth und/oder NFC-Schnittstelle ausgebildet ist.

Wie in Fig. 1 schematisch angedeutet ist, erzeugt der Schnittstellenschaltkreis 20 ein Abfragesignal 30, das hier über die Antenne 24 in die Mundhöhle 12 des Benutzers ausgesendet wird. Die Sensorelemente 18 besitzen jeweils einen - vorzugsweise passiven - Transponder, der in der Lage ist, das Abfragesignal 30 zu empfangen und in Abhängigkeit davon ein individuell codiertes Antwortsignal 32, 34 zu erzeugen. Beispielhaft ist ein erster individueller Code bei der Bezugsziffer 32' dargestellt und ein davon verschiedener zweiter individueller Code ist bei der Bezugsziffer 34' dargestellt. Das Antwortsignal 32 mit dem individuellen Code 32' wird von einem Sensorelement 18' erzeugt, das an dem Zahn 16' angeordnet ist. Das Antwortsignal 34 mit dem individuellen Code 34' wird von einem Sensorelement erzeugt, das an dem Zahn 16" angeordnet ist. Aufgrund der unterschiedlichen individuellen Codierungen 32', 34' kann der Schnittstellenschaltkreis 20 erkennen, von welchem der Sensorelemente 18 in der Mundhöhle 12 des Benutzers ein jeweiliges Antwortsignal 32, 34 stammt.

In der in Fig. 1 dargestellten Situation berührt die Zungenspitze der Zunge 14 die Zahninnenseite des Zahns 16', an der das Sensorelement 18' angeordnet ist. Dementsprechend bedeckt die Zunge 14 hier das Sensorelement 18', was zur Folge hat, dass das Antwortsignal 32 im Vergleich zu dem Antwortsignal 34 gedämpft ist oder sogar gänzlich unterdrückt wird. Infolgedessen empfängt der Schnittstellenschaltkreis 20 über die Antenne 24 das Antwortsignal 34 des Sensorelements 18" am Zahn 16" deutlich stärker als das Antwortsignal 32 von dem Zahn 16'. Gleiches gilt hier für weitere Antwortsignale (hier nicht dargestellt) von den weiteren Sensorelementen 18 in der Mundhöhle 12 des Benutzers. Durch Analyse der Antwortsignale von allen Sensorelementen 18 in der Mundhöhle 12 sowie Vergleich mit einer Erwartungshaltung, die beispielsweise in einem internen Speicher des Schnittstellenschaltkreises 12 hinterlegt sein kann, kann der Schnittstellenschaltkreis 20 erkennen, dass die Zungenspitze am Zahn 16' anliegt. In den bevorzugten Ausführungsbeispielen interpretiert der Schnittstellenschaltkreis 20 dies als eine "Betätigung" des Sensorelements 18' am Zahn 16' und er bestimmt in Abhängigkeit von dieser Betätigung ein Steuersignal für das technische Gerät. In einigen Ausführungsbeispielen kann das Steuersignal der Betätigung einer Taste auf einer handelsüblichen Computertastatur entsprechen, so dass der Benutzer Tastatureingaben mit seiner Zunge erzeugen kann, die mit den Tastatureingaben einer handelsüblichen Computertastatur kompatibel sind. In weiteren Ausführungsbeispielen kann die Betätigung des Sensorelements 18' beispielsweise den elektrischen Antrieb 26 einschalten oder ausschalten.

In bevorzugten Ausführungsbeispielen beinhaltet die Eingabevorrichtung 10 einen oder mehrere Kieferpositionssensoren 36, mit denen die Position des Unterkiefers des Benutzers relativ zum Oberkiefer und/oder ein Druck, mit dem der Benutzer den Unterkiefer und den Oberkiefer zusammenpresst, detektieren kann. Der Kieferpositionssensor kann auch außerhalb der Mundhöhle 12 angeordnet sein, insbesondere auf der Haut des Benutzers im Bereich des Discus Articularis. Der Kieferpositionssensor kann beispielsweise an dem Bügel 22 befestigt sein. In bevorzugten Ausführungsbeispielen erzeugt der Kieferpositionssensor 36 ein weiteres individuell codiertes Antwortsignal 38 in Reaktion auf das Abfragesignal 30. Der Schnittstellenschaltkreis 20 kann mit Hilfe des Kieferpositionssensors 36 eine momentane Kieferposition des Benutzers detektieren und in Abhängigkeit davon verschiedene Steuersignale für das technische Gerät erzeugen. In einigen Ausführungsbeispielen kann der Schnittstellenschaltkreis 20 das Kieferpositionssignal 38 nach Art einer Umschalttaste oder Auswahltaste logisch mit der Betätigung eines Sensorelements 18 durch die Zunge 14 kombinieren, so dass der Benutzer durch Variieren seiner Kieferposition verschiedene "Zeichen" mit gleicher Zungenposition oder Zungenbewegung erzeugen kann.

In bevorzugten Ausführungsbeispielen ist der Schnittstellenschaltkreis 20 dazu eingerichtet, eine zeitliche Abfolge von Zungenpositionen zu bestimmen, um auf diese Weise eine Zungenbewegung innerhalb der Mundhöhle 12 zu detektieren. In einigen bevorzugten Ausführungsbeispielen kann der Benutzer mit einer kontrollierten Zungenbewegung eine Interaktion mit dem technischen Gerät bewirken, wie etwa eine Bewegung des technischen Gerätes in eine Bewegungsrichtung, die der Bewegungsrichtung der Zunge 14 entspricht.

In bevorzugten Ausführungsbeispielen analysiert der Schnittstellenschaltkreis 20 mehrfache Betätigungen von Sensorelementen 18 innerhalb einer definierten Zeitspanne, um insbesondere zeitgleiche oder nahezu zeitgleiche Betätigungen von Sensorelementen 18 zu erkennen. In einigen bevorzugten Ausführungsbeispielen kann eine Mehrfachbetätigung von Sensorelementen 18 mit der Zunge 14 eine gezielte Interaktion mit dem technischen Gerät bewirken, ähnlich wie etwa die zeitgleiche Betätigung von mehreren Tasten auf der Tastatur eines herkömmlichen Computers vordefinierte Aktionen auslösen kann. In anderen Ausführungsbeispielen kann der Schnittstellenschaltkreis 20 dazu eingerichtet sein, mehrfache Betätigungen von Sensorelementen 18 innerhalb der definierten Zeitspanne zu ignorieren oder gegen eine hinterlegte Erwartungshaltung für typische unkontrollierte Zungenbewegungen vergleichen, um ungewollte oder unkontrollierte Zungenbewegungen herauszufiltern.

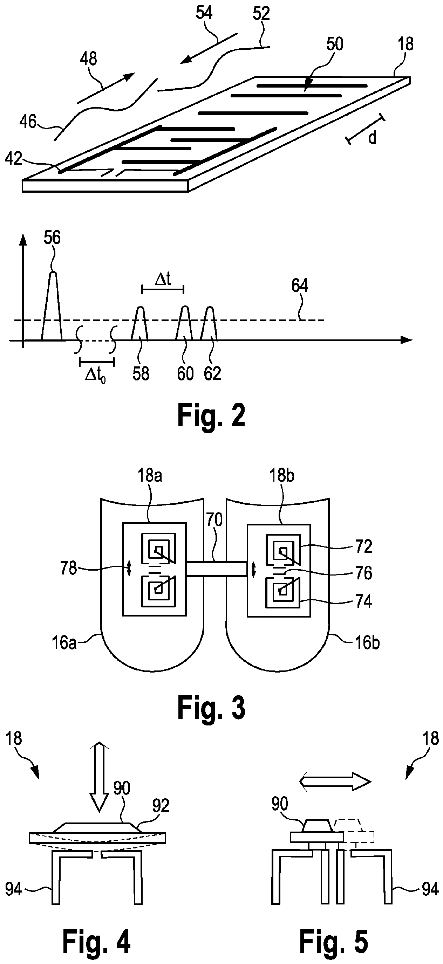

Fig. 2 zeigt ein bevorzugtes Ausführungsbeispiel für ein Sensorelement 18, hier allerdings in einer schematischen Darstellung ohne biokompatible Hülle.

Das Sensorelement 18 besitzt hier einen Körper 40, der beispielsweise aus einem kristallinen Halbleitermaterial, wie beispielsweise Lithiumniobat LiNbO3 hergestellt sein kann. Der Körper kann in diesem Fall sehr dünn sein und eine Dicke von beispielsweise kleiner oder gleich 0,2mm aufweisen. Auf dem Körper 40 sind Leiterstrukturen ausgebildet, beispielsweise als metallische Bahnen. Eine erste Leiterstruktur 42 bildet einen Wellenwandler (Interdigitalwandler) mit einer Empfangsantenne 44, über die das elektrische, magnetische und/oder elektromagnetische Abfragesignal 30 empfangen werden kann. Ein elektrisches Abfragesignal kann im Rahmen einer kapazitiven Kopplung zwischen den Sensorelementen und dem Schnittstellenschaltkreise verwendet sein. Ein magnetisches Abfragesignal kann bei einer induktiven Kopplung zwischen den Sensorelementen und dem Schnittstellenschaltkreise verwendet sein. Ein elektromagnetisches Abfragesignal 30 entspricht einem Funksignal. Prinzipiell erscheinen alle drei Varianten möglich, wobei ein elektromagnetisches Abfragesignal 30 in Bezug auf die nachfolgend erläuterte Funktion des Sensorelements 18 vorteilhaft erscheint.

Die Leiterstruktur 42 wandelt das Abfragesignal 30 in eine akustische Oberflächenwelle um, die in Fig. 2 bei der Bezugsziffer 46 angedeutet ist. Die Oberflächenwelle 46 breitet sich auf der Oberfläche des Körpers 40 in Richtung des Pfeils 48 aus. Bei der Bezugsziffer 50 ist eine weitere Leiterstruktur dargestellt, die auf der Oberfläche des Körpers 40 ausgebildet ist. Die weitere Leiterstruktur 50 beinhaltet hier im Wesentlichen mehrere Reflektoren, an denen die Oberflächenwelle 46 reflektiert wird. An jedem Reflektor der Leiterstruktur 50 entsteht folglich eine reflektierte Oberflächenwelle 52, die in Richtung des Pfeils 54 läuft und die Leiterstruktur 42 erreicht. Dort wird die zurücklaufende Oberflächenwelle 52 wieder in eine elektromagnetische Welle umgewandelt und über die Antenne 44 abgestrahlt.

Die Leiterstruktur 42 und die Antenne 44 sind vorzugsweise aneinander angepasst, mit hoher Impedanz und vorzugsweise direkt gekoppelt, um eine vorteilhafte Miniaturisierung des Sensorelements 18 zu ermöglichen. In einigen Ausführungsbeispielen besitzt das Sensorelement 18 eine Flächenausdehnung von etwa 2mm x 2mm.

Im unteren Teil der Fig. 2 ist ein schematisches Zeitdiagramm dargestellt, das bei der Bezugsziffer 56 einen Impuls zeigt, der das Abfragesignal 30 symbolisiert. Der Impuls 56 regt in der ersten Leiterstruktur 42 eine hinlaufende Oberflächenwelle 46 an. Bei den Bezugsziffern 58, 60, 62 sind hier drei kleinere Impulse dargestellt, die zeitlich aufeinander folgen und mit einer gewissen zeitlichen Verzögerung Δt₀ nach dem Impuls 56 auftreten. Die Impulse 58, 60 sind hier mit einem zeitlichen Abstand Δt dargestellt, der den räumlichen Abstand d zwischen den ersten beiden Reflektoren der zweiten Leiterstruktur 50 in Laufrichtung 48 der hinlaufenden Oberflächenwelle 46 entspricht. Mit anderen Worten erzeugt das Sensorelement 18 hier also auf einen Abfrageimpuls 56 hin eine Anzahl an zeitlich gestaffelten Antwortimpulsen 58, 60, 62, wobei der zeitliche Abstand der Antwortimpulse 58, 60, 62 zu dem Abfrageimpuls 56 und relativ zueinander der räumlichen Anordnung und Entfernung der Reflektoren in der Leiterstruktur 50 relativ zu der Leiterstruktur 42 entspricht. Die Signalstärke der Antwortimpulse 58, 60, 62, die von der ersten Leiterstruktur 42 als elektromagnetische Welle ausgesendet werden und dann bei der Antenne 24 des Schnittstellenschaltkreises 20 ankommen, ist üblicherweise deutlich geringer als die Signalstärke des Abfrageimpulses 56, wie dies in Fig. 2 vereinfacht dargestellt ist, da sich die Energie des Abfrageimpulses 56 auf mehrere Antwortimpulse 58, 60, 62 verteilt. Der zeitliche Abstand Δt₀ der Antwortimpulse 58, 60, 62 zum Abfragesignal 56 ist aufgrund der Ausbreitungsgeschwindigkeit der akustischen Oberflächenwellen 46, 52 wesentlich größer als der Zeitversatz zu Rückstreusignalen bzw. Signalechos des Abfragesignals 56 aufgrund von Reflexionen in der Mundhöhle. Der zeitliche Abstand Δt₀ vereinfacht die Auswertung der Antwortimpulse 60, 62 im Schnittstellenschaltkreis 20.

Bei der Bezugsziffer 64 ist eine Schwellenwertlinie angedeutet, die eine Signalschwelle repräsentiert, die in dem Schnittstellenschaltkreis 20 hinterlegt ist. Nur wenn eine Folge von Antwortimpulsen 58, 60, 62 die durch die Linie 64 repräsentierte Signalschwelle überschreitet, erkennt der Schnittstellenschaltkreis 20 in diesem Ausführungsbeispiel die entsprechende Pulsfolge als Antwortsignal des Sensorelements 18. Der zeitliche Abstand Δt der einzelnen Antwortimpulse in der Impulsfolge 58, 60, 62 repräsentiert eine individuelle Codierung, die das entsprechende Sensorelement 18 eindeutig identifiziert. Fig. 2 zeigt daher in einer vereinfachten Darstellung einen sog. SAW-Tag sowie eine Illustration einer vorteilhaften Funktionsweise. SAW-Tags gibt es seit den 1960er Jahren und sie bilden gewissermaßen einen geometrisch codierten Transponder, der sich für die Realisierung der neuen Eingabevorrichtung besonders gut eignet, weil SAW-Tags als passive Transponder sehr kleinbauend und kostengünstig hergestellt werden können. Wie bereits weiter oben angedeutet, kann der Benutzer mit Hilfe seiner Zunge die Signalstärke der elektromagnetischen Welle, die einen SAW-Tag als Abfragesignal 30 erreicht, sowie die Signalstärke etwaiger Antwortimpulse 58, 60, 62 dämpfen und somit dazu beitragen, dass der Schnittstellenschaltkreis 20 das Antwortsignal 32 von dem Sensorelement 18' (siehe Fig. 1) nicht oder zumindest nicht mit ausreichender Signalstärke erhält.

Fig. 3 zeigt ein weiteres Ausführungsbeispiel anhand von zwei Sensorelementen 18a, 18b, die an jeweils einem Zahn 16a, 16b eines Benutzers angeordnet sein können. Die Sensorelemente 18a, 18b können mit Hilfe von SAW-Tags entsprechend Fig. 2 und/oder mit Hilfe einer anderen Transpondertechnologie realisiert sein.

In dem Ausführungsbeispiel gemäß Fig. 3 sind zwei benachbarte Sensorelemente 18a, 18b an einem gemeinsamen Sensorträger 70 gehalten. Wie weiter oben schon angedeutet, kann der Sensorträger 70 aus Silikon, Kunststoff oder Metall hergestellt sein und je nach Anwendung ein oder mehrere Sensorelemente 18 an den Zähnen eines Benutzers fixieren. Geeignete Sensorträger 70 sind aus dem Bereich der Zahnmedizin und Kieferchirurgie prinzipiell bekannt.

Eine Besonderheit des Ausführungsbeispiels gemäß Fig. 3 liegt darin, dass hier jedes der Sensorelemente 18a, 18b zwei Sende-/Empfangsantennen 72, 74 beinhaltet, die räumlich nebeneinander angeordnet sind. Jede der beiden Sensorantennen 72, 74 ist hier "offen", d.h. die elektrische Leiterbahn der jeweiligen Antenne 72, 74 ist unterbrochen. Zwischen den beiden Antennen 72, 74 ist hier ein Schalter 76 mit zwei Kontaktpfaden angeordnet. Der Schalter 76 kann mit der Zunge in Richtung des Doppelpfeils 78 verschoben werden, um wahlweise die eine Antenne 72 oder die andere Antenne 74 zu schließen. Jede der beiden Antennen 72, 74 eines Sensorelements 18a, 18b kann mit einem Speicherchip gekoppelt sein, in dem eine individuelle Codierung gespeichert ist. Der Benutzer kann daher mit Hilfe seiner Zunge die Sensorelemente 18a, 18b wahlweise aktivieren und dabei auswählen, welche der jeweiligen Codierungen auf ein Abfragesignal 30 hin an den Schnittstellenschaltkreis 20 gesendet werden soll. Mit einem derartigen Ausführungsbeispiel ist es möglich, dass ein Sensorelement 18a, 18b wahlweise verschiedene individuelle Codierungen aussendet und somit verschiedene Betätigungen zu dem Schnittstellenschaltkreis 20 überträgt. Außerdem kann mit einem Ausführungsbeispiel gemäß Fig. 3 die Bewegungsrichtung der Zunge und ggf. eine oszillierende Bewegung der Zunge auf einfache Weise detektiert werden.

Fig. 4 zeigt in einer vereinfachten Darstellung ein Sensorelement 18 mit einer Kontaktfläche 90, die hier auf einer Erhebung 92 ausgebildet ist. Die Kontaktfläche 90 kann hier durch Druck senkrecht zur Kontaktfläche 90 auf eine Leiterbahnstruktur 94 gedrückt werden, um einen elektrischen Kontakt zu schließen. Fig. 5 zeigt ein weiteres Ausführungsbeispiel eines Sensorelements mit einem Schalter, der hier parallel zu der Kontaktfläche 90 verschoben werden muss, um eine doppelte Leiterstruktur 94 selektiv zu öffnen oder zu schließen. Die Fig. 4 und 5 zeigen somit mögliche Realisierungen für einen Schalter 76 gemäß Fig. 3.

In allen bevorzugten Ausführungsbeispielen ermöglicht die natürliche Bewegung der Zunge 14 des Benutzers über die in das Innere der Mundhöhle 12 weisenden Zahnoberflächen eine weitgehend ermüdungsfreie Bedienung der Eingabevorrichtung 10. Die Anordnung eines jeweils einzelnen Sensorelements 18 an einer Zahnfläche ermöglicht eine gezielte Daten- und/oder Steuersignalerzeugung mit einer geringen Fehlerrate aufgrund der feinmotorischen Fähigkeiten bzw. des feinmotorischen Gedächtnisses der Zunge 14 innerhalb der Mundhöhle 12. Eine Anordnung der Sensorelemente an den Zahninnenflächen trägt vorteilhaft zu einer ermüdungsfreien Bedienung bei. Besonders vorteilhaft ist eine kombinierte Auswertung der Antwortsignale von mehreren Sensorelementen, ggf. in Kombination mit einem oder mehreren Signalen, die eine Kieferposition des Benutzers repräsentieren, da auf diese Weise eine sehr hohe Anzahl an verschiedenen Eingabedaten/-Befehlen voneinander unterschieden werden kann. Somit ermöglichen Ausführungsbeispiele der neuen Eingabevorrichtung die Realisierung einer Tastaturfunktion, wie sie von herkömmlichen Computertastaturen bekannt ist, jedoch im Unterschied zu einer manuellen Betätigung hier mit Zungenbetätigung.

## Patentansprüche

1. Eingabevorrichtung zur Bedienung und/oder Steuerung eines technischen Gerätes (26, 28) durch einen Benutzer, mit einer Vielzahl von Sensorelementen (18), die dazu eingerichtet sind, räumlich verteilt in der Mundhöhle (12) des Benutzers angeordnet zu werden, um eine jeweils momentane Zungenposition und/oder Zungenbewegung des Benutzers zu detektieren, und mit einem Schnittstellenschaltkreis (20), der mit den Sensorelementen (18) gekoppelt ist und der dazu eingerichtet ist, in Abhängigkeit von den Sensorelementen (18) eine Vielzahl von Steuersignalen zu erzeugen und an das technische Gerät (26, 28) zu übertragen, **dadurch gekennzeichnet, dass** die Sensorelemente (18) jeweils einen Transponder beinhalten, der dazu eingerichtet ist, ein Abfragesignal (30) aufzunehmen und in Abhängigkeit davon ein individuell codiertes Antwortsignal (32, 34) an den Schnittstellenschaltkreis (20) zu übertragen, wobei der Schnittstellenschaltkreis (20) dazu eingerichtet ist, das Abfragesignal (30) zu erzeugen.

2. Eingabevorrichtung nach Anspruch 1, **dadurch gekennzeichnet, dass** die Sensorelemente (18) jeweils an einem Sensorträger (70) angeordnet sind, der dazu eingerichtet ist, die Sensorelemente (18) an den Zähnen (16) des Benutzers zu platzieren.

3. Eingabevorrichtung nach Anspruch 1 oder 2, **gekennzeichnet durch** einen Halter (22), der dazu eingerichtet ist, den Schnittstellenschaltkreis (20) außerhalb der Mundhöhle (12) zu halten.

4. Eingabevorrichtung nach einem der Ansprüche 1 bis 3, **dadurch gekennzeichnet, dass** die Sensorelemente (18) jeweils einen SAW-Tag als Transponder beinhalten.

5. Eingabevorrichtung nach einem der Ansprüche 1 bis 4, **dadurch gekennzeichnet, dass** der Schnittstellenschaltkreis (20) dazu eingerichtet ist, eine Vielzahl von individuell codierten Antwortsignalen (32, 34) in Bezug auf deren jeweilige Signalstärke zu analysieren.

6. Eingabevorrichtung nach Anspruch 4, **dadurch gekennzeichnet, dass** der Schnittstellenschaltkreis (20) dazu eingerichtet ist, die Signalstärke einer Vielzahl von Antwortsignalen (32, 34) mit einem Erwartungswert zu vergleichen.

7. Eingabevorrichtung nach einem der Ansprüche 1 bis 6, **dadurch gekennzeichnet, dass** der Schnittstellenschaltkreis (20) dazu eingerichtet ist, eine Vielzahl von Antwortsignalen (32, 34) unterhalb eines definierten Schwellenwertes (64) nicht bezüglich der Signalstärke auszuwerten.

8. Eingabevorrichtung nach einem der Ansprüche 1 bis 7, **dadurch gekennzeichnet, dass** der Schnittstellenschaltkreis (20) dazu eingerichtet ist, eine Vielzahl von individuell codierten Antwortsignalen (32, 34) logisch miteinander zu verknüpfen.

9. Eingabevorrichtung nach einem der Ansprüche 1 bis 8, **dadurch gekennzeichnet, dass** die Sensorelemente (18) jeweils einen Schalter (76) beinhalten, der dazu eingerichtet ist, den Transponder wahlweise zu aktivieren oder zu deaktivieren.

10. Eingabevorrichtung nach Anspruch 9, **dadurch gekennzeichnet, dass** die Sensorelemente (90) jeweils eine definierte Kontaktfläche für die Zunge (14) des Benutzers aufweisen, wobei der Schalter senkrecht zu der Kontaktfläche (90) betätigt werden kann.

11. Eingabevorrichtung nach Anspruch 9 oder 10, **dadurch gekennzeichnet, dass** die Sensorelemente jeweils eine definierte Kontaktfläche (90) für die Zunge (14) des Benutzers aufweisen, wobei der Schalter parallel zu der Kontaktfläche (90) betätigt werden kann.

12. Eingabevorrichtung nach einem der Ansprüche 1 bis 11, **dadurch gekennzeichnet, dass** die Sensorelemente (18) jeweils eine Erhebung (92) und/oder Vertiefung aufweisen, die der Zunge (14) des Benutzers eine tastbare Markierung bietet.

13. Eingabevorrichtung nach einem der Ansprüche 1 bis 12, **gekennzeichnet durch** einen Kieferpositionssensor (36), der dazu eingerichtet ist, eine Kieferposition des Benutzers zu detektieren, wobei der Schnittstellenschaltkreis (20) dazu eingerichtet ist, die Vielzahl von Steuersignalen ferner in Abhängigkeit von dem Kieferpositionssensor (36) zu erzeugen.

14. Eingabevorrichtung nach einem der Ansprüche 1 bis 13, **dadurch gekennzeichnet, dass** die Sensorelemente (18) eine geschlossene biokompatible Hülle aufweisen.

15. Verfahren zur Bedienung und/oder Steuerung eines technischen Gerätes (26, 28) durch einen Benutzer, mit den Schritten:
- Bereitstellen einer Vielzahl von Sensorelementen (18) räumlich verteilt in der Mundhöhle (12) des Benutzers,
- Detektieren einer jeweils momentanen Zungenposition und/oder Zungenbewegung des Benutzers unter Verwendung der Sensorelemente (18),
- Erzeugen einer Vielzahl von Steuersignalen in Abhängigkeit von der jeweils momentanen Zungenposition und/oder Zungenbewegung des Benutzers mit Hilfe eines Schnittstellenschaltkreises (20), und
- Übertragen der Vielzahl von Steuersignalen von dem Schnittstellenschaltkreis an das technische Gerät (26, 28),
**dadurch gekennzeichnet, dass** der Schnittstellenschaltkreis (20) ein Abfragesignal (30) erzeugt, und dass die Sensorelemente (18) jeweils einen Transponder beinhalten, der das Abfragesignal (30) aufnimmt und ein individuell codiertes Antwortsignal (32, 34) in Abhängigkeit von der jeweils momentanen Zungenposition und/oder Zungenbewegung des Benutzers an den Schnittstellenschaltkreis (20) überträgt.

## Claims

1. An input apparatus for operating and/or controlling a technical device (26, 28) by a user, comprising a plurality of sensor elements (18) which are configured to be arranged spatially distributed in the oral cavity (12) of the user in order to detect a respective current tongue position and/or tongue movement of the user, and comprising an interface circuit (20) which is coupled to the sensor elements (18) and which is configured to generate a plurality of control signals depending on the sensor elements (18) and to transmit them to the technical device (26, 28), **characterized in that** the sensor elements (18) each include a transponder which is configured to receive an interrogation signal (30) and to transmit an individually encoded response signal (32, 34) to the interface circuit (20) in response thereto, wherein the interface circuit (20) is configured to generate the interrogation signal (30).

2. The input apparatus according to claim 1, **characterized in that** the sensor elements (18) each are arranged on a sensor carrier (70) which is configured to place the sensor elements (18) on the user's teeth (16).

3. The input apparatus according to claim 1 or 2, **characterized by** a holder (22) configured to hold the interface circuit (20) outside the oral cavity (12).

4. The input apparatus according to any one of claims 1 to 3, **characterized in that** the sensor elements (18) each contain a SAW tag as a transponder.

5. The input apparatus according to any one of claims 1 to 4, **characterized in that** the interface circuit (20) is configured to analyze a plurality of individually encoded response signals (32, 34) with respect to their respective signal strength.

6. The input apparatus according to claim 4, **characterized in that** the interface circuit (20) is configured to compare the signal strength of a plurality of response signals (32, 34) with an expected value.

7. The input apparatus according to any one of claims 1 to 6, **characterized in that** the interface circuit (20) is configured not to evaluate a plurality of response signals (32, 34) below a defined threshold (64) with respect to signal strength.

8. The input apparatus according to any one of claims 1 to 7, **characterized in that** the interface circuit (20) is configured to logically interlink a plurality of individually encoded response signals (32, 34).

9. The input apparatus according to any one of claims 1 to 8, **characterized in that** the sensor elements (18) each include a switch (76) which is configured to selectively activate or deactivate the transponder.

10. The input apparatus according to claim 9, **characterized in that** the sensor elements (90) each have a defined contact surface for the tongue (14) of the user, wherein the switch can be actuated perpendicular to the contact surface (90).

11. The input apparatus according to claim 9 or 10, **characterized in that** the sensor elements each have a defined contact surface (90) for the tongue (14) of the user, wherein the switch can be actuated parallel to the contact surface (90).

12. The input apparatus according to any one of claims 1 to 11, **characterized in that** the sensor elements (18) each have a protrusion (92) and/or indentation that provides a tactile marker for the user's tongue (14).

13. The input apparatus according to any one of claims 1 to 12, **characterized by** a jaw position sensor (36) configured to detect a jaw position of the user, wherein the interface circuit (20) is configured to generate the plurality of control signals further depending on the jaw position sensor (36).

14. The input apparatus according to any one of claims 1 to 13, **characterized in that** the sensor elements (18) have a closed biocompatible shell.

15. A method for operating and/or controlling a technical device (26, 28) by a user, comprising the steps:
- providing a plurality of sensor elements (18) spatially distributed in the oral cavity (12) of the user,
- detecting a respective current tongue position and/or tongue movement of the user using the sensor elements (18),
- generating a plurality of control signals depending on the respective current tongue position and/or tongue movement of the user by means of an interface circuit (20), and
- transmitting the plurality of control signals from the interface circuit to the technical device (26, 28),
**characterized in that** the interface circuit (20) generates an interrogation signal (30), and the sensor elements (18) each include a transponder that receives the interrogation signal (30) and transmits an individually encoded response signal (32, 34) to the interface circuit (20) depending on the respective current tongue position and/or tongue movement of the user.

## Revendications

1. Périphérique d'entrée pour l'utilisation et/ou la commande d'un appareil technique (26, 28) par un utilisateur, comprenant une pluralité d'éléments capteurs (18), qui sont conçus pour être répartis spatialement dans la cavité buccale (12) de l'utilisateur afin de détecter une position de la langue et/ou un mouvement de la langue, respectivement momentané, de l'utilisateur, et comprenant un circuit d'interface (20), lequel est couplé aux éléments capteurs (18) et conçu pour générer une pluralité de signaux de commande en fonction des éléments capteurs (18) et les transmettre à l'appareil technique (26, 28), **caractérisé en ce que** les éléments capteurs (18) contiennent chacun un transpondeur, lequel est conçu pour recevoir un signal d'interrogation (30) et pour transmettre, en fonction de celui-ci, un signal de réponse (32, 34) codé individuellement au circuit d'interface (20), le circuit d'interface (20) étant conçu pour générer le signal d'interrogation (30).

2. Périphérique d'entrée selon la revendication 1, **caractérisé en ce que** les éléments capteurs (18) sont respectivement disposés sur un porte-capteur (70), lequel est conçu pour placer les éléments capteurs (18) au niveau des dents (16) de l'utilisateur.

3. Périphérique d'entrée selon la revendication 1 ou 2, **caractérisé par** un élément de maintien (22), lequel est conçu pour maintenir le circuit d'interface (20) à l'extérieur de la cavité buccale (12).

4. Périphérique d'entrée selon l'une des revendications 1 à 3, **caractérisé en ce que** les éléments capteurs (18) contiennent chacun une étiquette SAW en tant que transpondeur.

5. Périphérique d'entrée selon l'une des revendications 1 à 4, **caractérisé en ce que** le circuit d'interface (20) est conçu pour analyser une pluralité de signaux de réponse (32, 34) codés individuellement en ce qui concerne leur intensité de signal respective.

6. Périphérique d'entrée selon la revendication 4, **caractérisé en ce que** le circuit d'interface (20) est conçu pour comparer l'intensité de signal d'une pluralité de signaux de réponse (32, 34) à une valeur attendue.

7. Périphérique d'entrée selon l'une des revendications 1 à 6, **caractérisé en ce que** le circuit d'interface (20) est conçu pour ne pas évaluer une pluralité de signaux de réponse (32, 34) en dessous d'une valeur de seuil définie (64) en ce qui concerne leur intensité de signal.

8. Périphérique d'entrée selon l'une des revendications 1 à 7, **caractérisé en ce que** le circuit d'interface (20) est conçu pour combiner logiquement entre eux une pluralité de signaux de réponse (32, 34) codés individuellement.

9. Périphérique d'entrée selon l'une des revendications 1 à 8, **caractérisé en ce que** les éléments capteurs (18) comprennent respectivement un commutateur (76), lequel est conçu pour activer ou désactiver sélectivement le transpondeur.

10. Périphérique d'entrée selon la revendication 9, **caractérisé en ce que** les éléments capteurs (90) possèdent respectivement une surface de contact définie pour la langue (14) de l'utilisateur, le commutateur pouvant être actionné perpendiculairement à la surface de contact (90).

11. Périphérique d'entrée selon la revendication 9 ou 10, **caractérisé en ce que** les éléments capteurs possèdent respectivement une surface de contact (90) définie pour la langue (14) de l'utilisateur, le commutateur pouvant être actionné parallèlement à la surface de contact (90).

12. Périphérique d'entrée selon l'une des revendications 1 à 11, **caractérisé en ce que** les éléments capteurs (18) possèdent respectivement un bossage (92) et/ou une empreinte qui offre un repère tactile à la langue (14) de l'utilisateur.

13. Périphérique d'entrée selon l'une des revendications 1 à 12, **caractérisé par** un capteur de position de mâchoire (36), lequel est conçu pour détecter une position de mâchoire de l'utilisateur, le circuit d'interface (20) étant conçu pour générer la pluralité de signaux de commande en outre en fonction du capteur de position de mâchoire (36).

14. Périphérique d'entrée selon l'une des revendications 1 à 13, **caractérisé en ce que** les éléments capteurs (18) possèdent une enveloppe biocompatible fermée.

15. Procédé d'utilisation et/ou de commande d'un appareil technique (26, 28) par un utilisateur, comprenant les étapes suivantes :
- fourniture d'une pluralité d'éléments capteurs (18) répartis spatialement dans la cavité buccale (12) de l'utilisateur,
- détection d'une position de la langue et/ou d'un mouvement de la langue, respectivement momentané, de l'utilisateur en utilisant les éléments capteurs (18),
- génération d'une pluralité de signaux de commande en fonction de la position de la langue et/ou du mouvement de la langue, respectivement momentané, de l'utilisateur à l'aide d'un circuit d'interface (20), et
- transmission de la pluralité de signaux de commande du circuit d'interface à l'appareil technique (26, 28),
**caractérisé en ce que** le circuit d'interface (20) génère un signal d'interrogation (30), et **en ce que** les éléments capteurs (18) contiennent respectivement un transpondeur qui reçoit le signal d'interrogation (30) et transmet au circuit d'interface (20) un signal de réponse (32, 34) codé individuellement en fonction de la position de la langue et/ou du mouvement de la langue, respectivement momentané, de l'utilisateur.
